# EUROPEAN PATENT APPLICATION

(11) **EP 2 100 608 A1**
(43) Date of publication of application: **16.09.2009**
(21) Application number: 07850165.7
(22) Date of filing: 06.12.2007
(51) Int. Cl.: A61K 31/4365, A61K 9/20, A61P 7/02

(54) **METHOD FOR PRODUCING SOLID PREPARATION**

(30) Priority: 07.12.2006 JP 2006330373
(71) Applicant: Daiichi Sankyo Company, Limited, Chuo-ku Tokyo 103-8426 (JP); Ube Industries, Ltd., Ube-shi Yamaguchi 755-8633 (JP)
(72) Inventor: WATANABE, Tomoyuki, Kanagawa 254-0014 (JP)
(74) Representative: Fairbairn, Angus Chisholm
(86) International application number: PCT/JP2007/073549
(87) International publication number: WO 2008/072533

(57) **Abstract**

Disclosed is a method for producing a solid preparation containing a compound represented by the general formula (I) below or a pharmacologically acceptable salt thereof and having improved dissolvability. Specifically disclosed is a method for producing a solid preparation containing a compound represented by the general formula (I) below or a pharmacologically acceptable salt thereof, which comprises a step wherein a composition containing a compound represented by the general formula (I) below or a pharmacologically acceptable salt thereof is mixed, while applying mechanical stress to the composition.

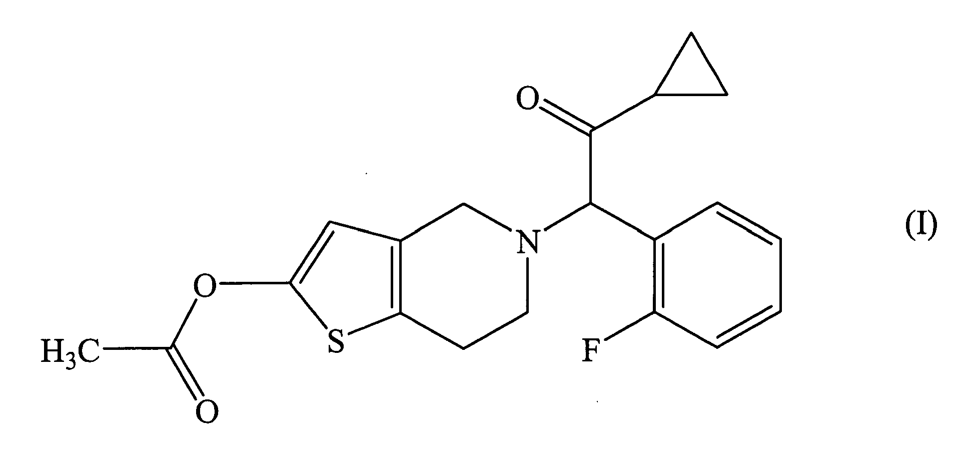

## Description

### [TECHNICAL FIELD]

The present invention relates to a method for producing a solid preparation containing a compound represented by the following general formula (I): 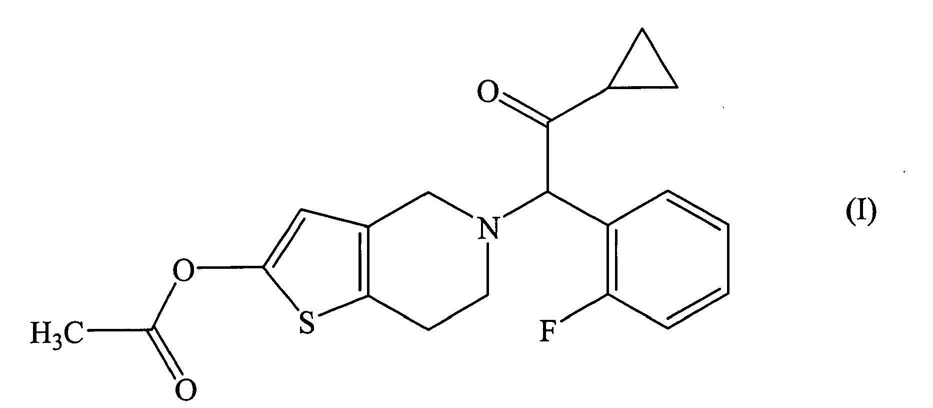 or a pharmacologically acceptable salt thereof, which includes a step in which a composition containing the compound represented by the aforementioned general formula (I) or a pharmacologically acceptable salt thereof is mixed, while applying mechanical stress to the composition, and specifically to the method being a method for producing a solid preparation containing the compound represented by the aforementioned general formula (I) or a pharmacologically acceptable salt thereof, which includes a step in which a composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s. In addition, the present invention relates to a solid preparation containing the compound represented by the aforementioned general formula (I) or a pharmacologically acceptable salt thereof, which is produced by the aforementioned method and has excellent dissolvability.

### [BACKGROUND ART]

The compound represented by the aforementioned general formula (I) or a pharmacologically acceptable salt thereof is known as a compound having platelet aggregation inhibition activity (Patent Document 1 or 2).

Patent Document 1 discloses that the compound represented by the aforementioned general formula (I) or a pharmacologically acceptable salt thereof "can be administered as itself, or can be mixed with suitable carriers, fillers, diluents and the like that are pharmacologically acceptable, ............ administered orally or parenterally as a pharmaceutical composition".

Although Patent Documents 2 and 3 disclose a preparation example of the hydrochloride of the compound represented by the aforementioned general formula (I), both of the preparation examples disclose that the preparation is made by merely "mixing" the aforementioned compound and an additive.

Patent Documents 4, 5 and 6 disclose that the compound represented by the aforementioned general formula (I) or a pharmacologically acceptable salt thereof "can be administered as itself, or can be mixed with suitable carriers, fillers, diluents and the like that are pharmacologically acceptable, ............ administered orally or parenterally as a pharmaceutical composition", and a preparation example of the hydrochloride of the compound represented by the aforementioned general formula (I). However, both of the preparation examples disclose that the preparation is made by merely "mixing" the aforementioned compound and additives.

Therefore, none of the Patent Documents discloses or teaches that by applying mechanical stress to a composition of the compound represented by the aforementioned general formula (I) or a pharmacologically acceptable salt thereof in a step in which the composition is mixed, the finally obtained solid preparation can have excellent dissolvability. In addition, none of the Patent Documents discloses or teaches with respect to a composition of the compound represented by the aforementioned general formula (I) or a pharmacologically acceptable salt thereof that by using a convection mixer equipped with a stirring blade and rotating the stirring blade at a particular velocity, the finally obtained solid preparation can have excellent dissolvability.
[Patent Document 1] Japanese Patent Application (Kokai) No. Hei 6-41139
[Patent Document 2] Japanese Patent Application (Kokai) No. 2002-145883
[Patent Document 3] pamphlet of International Publication WO 2004/098713
[Patent Document 4] Japanese Patent Application (Kokai) No. 2002-255814
[Patent Document 5] Japanese Patent Application (Kokai) No. 2003-246735
[Patent Document 6] Japanese Patent Application (Kokai) No. 2004-51639

### [DISCLOSURE OF THE INVENTION]

### [PROBLEMS TO BE SOLVED BY THE INVENTION]

An object of the present invention is to provide a method for producing a solid preparation containing a compound represented by the aforementioned general formula (I) or a pharmacologically acceptable salt thereof, which includes a step in which a composition containing the compound represented by the aforementioned general formula (I) or a pharmacologically acceptable salt thereof is mixed, while applying mechanical stress to the composition, and specifically the method being a method for producing a solid preparation containing the compound represented by the aforementioned general formula (I) or a pharmacologically acceptable salt thereof, which includes a step in which a composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s. In addition, it is also an object of the present invention to provide a solid preparation containing the compound represented by the aforementioned general formula (I) or a pharmacologically acceptable salt thereof, which is produced by the aforementioned method and has excellent dissolvability.

### [MEANS FOR SOLVING THE PROBLEMS]

As a result of conducting extensive studies to solve the aforementioned problems, the inventors of the present invention found that a solid preparation containing the compound represented by the aforementioned general formula (I) or a pharmacologically acceptable salt thereof, which is produced by a method including a step in which a composition containing the compound represented by the aforementioned general formula (I) or a pharmacologically acceptable salt thereof is mixed while applying mechanical stress to the composition, can have excellent dissolvability; and in particular found that a solid preparation containing the compound represented by the aforementioned general formula (I) or a pharmacologically acceptable salt thereof, which is produced by a method including a step in which a composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s, can have excellent dissolvability, thereby leading to completion of the present invention.

That is, the present invention is:
(1) a method for producing a solid preparation containing a compound represented by the following general formula (I): 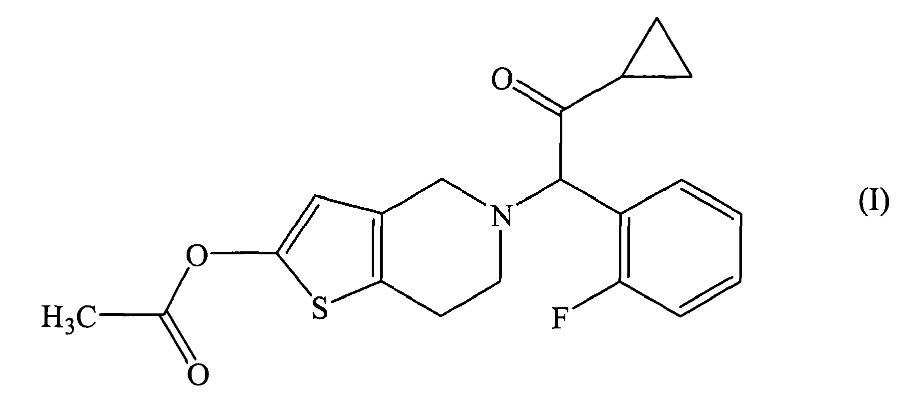 or a pharmacologically acceptable salt thereof, comprising a step in which a composition containing the compound represented by the aforementioned general formula (I) or a pharmacologically acceptable salt thereof is mixed, while applying mechanical stress to the composition, preferably,
(2) a method for producing a solid preparation containing the compound represented by the aforementioned general formula (I) or a pharmacologically acceptable salt thereof, comprising a step in which a composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s,
(3) the method according to (2), wherein the circumferential velocity at the end of the stirring blade is 5.0 m/s to 20 m/s,
(4) the solid preparation according to any one of (1) to (3), wherein the compound represented by the general formula (I) or a pharmacologically acceptable salt thereof is a compound represented by the following formula (Ia): 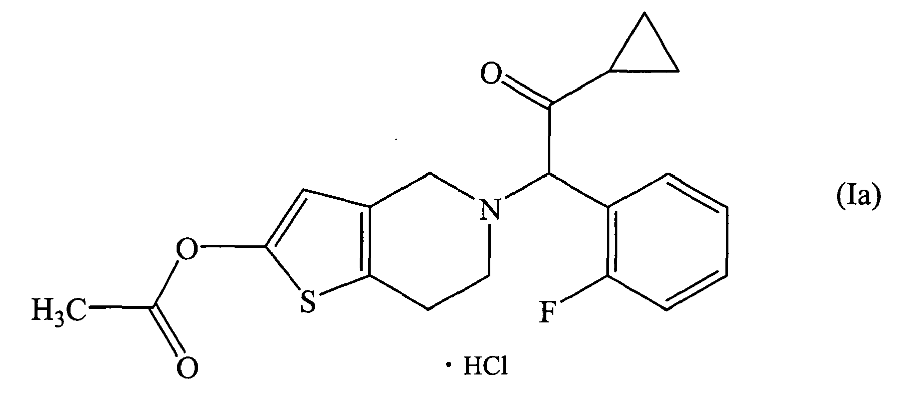 , or
(5) the method according to any one of (1) to (4), wherein the solid preparation is in the form of tablets.
   In addition, the present invention is:
(6) a solid preparation containing a compound represented by the following general formula (I): 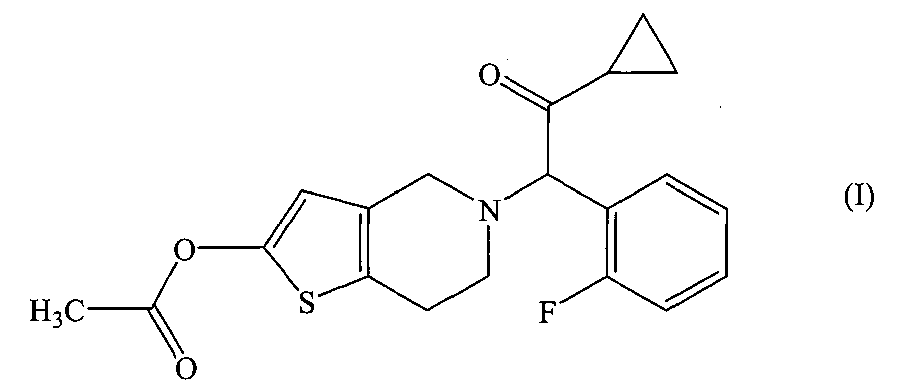 or a pharmacologically acceptable salt thereof, which is produced by a method comprising a step in which a composition containing the compound represented by the aforementioned general formula (I) or a pharmacologically acceptable salt thereof is mixed, while applying mechanical stress to the composition,
   preferably,
(7) a solid preparation containing the compound represented by the aforementioned general formula (I) or a pharmacologically acceptable salt thereof, which is produced by a method comprising a step in which a composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s,
(8) the solid preparation according to (7), wherein the circumferential velocity at the end of the stirring blade is 5.0 m/s to 20 m/s,
(9) the solid preparation according to any one of (6) to (8), wherein the compound represented by the general formula (I) or a pharmacologically acceptable salt thereof is a compound represented by the following formula (Ia): 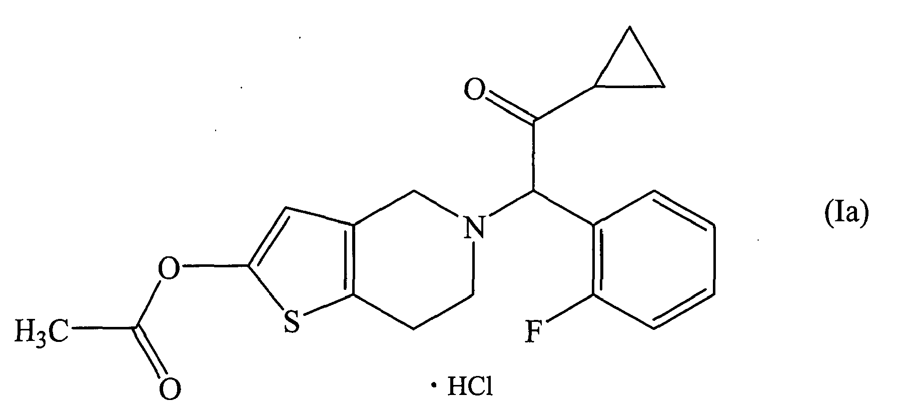 , or
(10) the solid preparation according to any one of (6) to (9), wherein the preparation is in the form of tablets.

### [EFFECT OF THE INVENTION]

According to the present invention, a solid preparation containing a compound represented by the aforementioned formula (I) or a pharmacologically acceptable salt thereof and having excellent dissolvability can be provided, which is produced by a method including a step in which a composition containing the compound represented by the aforementioned general formula (I) or a pharmacologically acceptable salt thereof is mixed while applying mechanical stress to the composition; in particular a solid preparation containing the compound represented by the aforementioned general formula (I) or a pharmacologically acceptable salt thereof and having excellent dissolvability can be provided, which is produced by a method including a step in which a composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s.

The solid preparation of the present invention is, for example, useful for the treatment and/or prophylaxis of thrombosis or embolism (preferably thrombosis) and the like (preferably is a drug for the treatment and/or prophylaxis of thrombosis).

### [BEST MODE FOR CARRYING OUT THE INVENTION]

The compound represented by the following general formula (I) : 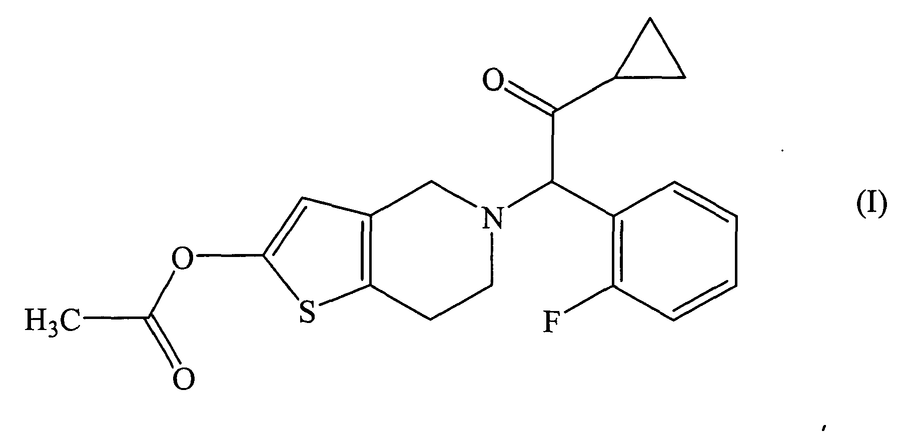 that is, 2-acetoxy-5-(α-cyclopropylcarbonyl-2-fluorobenzyl)-4,5,6,7-tetrahydrothieno[3,2-c]pyridine, or a pharmacologically acceptable salt thereof, which is the active ingredient of the solid preparation of the present invention, is disclosed in Japanese Patent Application (Kokai) No. Hei 6-41139 or Japanese Patent Application (Kokai) No. 2002-145883, and can be prepared accordingly.

As the "pharmacologically acceptable salt thereof" of the present invention, there may be mentioned for example, hydrohalides such as hydrofluoride, hydrochloride, hydrobromide or hydroiodide; inorganic acid salts such as nitrate, perchloric acid salt, sulfate or phosphate; lower-alkyl sulfonic acid salts such as methanesulfonate, trifluoromethanesulfonate or ethanesulfonate; aryl sulfonic acid salts such as benzenesulfonate or p-toluenesulfonate; organic acid salts such as acetate, malate, fumarate, succinate, citrate, ascorbate, tartrate, oxalate or maleate; or amino acid salts such as glycine salt, lysine salt, arginine salt, ornithine salt, glutamic acid salt or aspartic acid salt. The preferred salts are hydrohalides or organic acid salts, more preferably hydrochloride or maleate, and most preferably hydrochloride.

In the "step in which a composition is applied with mechanical stress" of the present invention, the mechanism for applying stress is not limited so long as the step can apply mechanical stress from the external to the composition containing the compound represented by the aforementioned general formula (I) or a pharmacologically acceptable salt thereof. For example, a step in which a composition is mixed or stirred while applying mechanical stress to the composition, a step in which the composition is granulated by compressing the composition before a step in which the composition is mixed, a pulverization step in which the composition is pulverized by mechanical stress or shear force, a step in which a tablet is compression-molded by a tableting step before the mixing step of the composition, and the like can be mentioned. The "step in which mechanical stress is applied to the composition" is preferably a step in which the composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s.

In the "step in which mechanical stress is applied to the composition", the amount of stress applied to the composition is not limited so long as it can improve the dissolvability of the active ingredient. Here, in the case where a convection mixer equipped with a stirring blade is used and the stirring blade is rotated, it is preferably stress obtained by rotating the stirring blade such that the circumferential velocity at the end of the stirring blade is 1.4 m/s to 40 m/s, more preferably stress obtained by rotating the stirring blade such that the circumferential velocity at the end of the stirring blade is 5.0 m/s to 40 m/s, and most preferably stress obtained by rotating the stirring blade such that the circumferential velocity at the end of the stirring blade is 5.0 m/s to 20 m/s. In other embodiments, it is preferably stress which corresponds to 20 N/mm² or larger, more preferably stress which corresponds to 40 to 600 N/mm², and most preferably stress which corresponds to 60 to 400 N/mm².

The solid preparation may further contain additives such as appropriate pharmacologically acceptable fillers, lubricants, binders, emulsifiers, stabilizers, corrigents and/or diluents.

As the "fillers" used, there may be mentioned for example, organic fillers including sugar derivatives such as lactose, sucrose, glucose, mannitol or sorbitol; starch derivatives such as corn starch, potato starch, α-starch or dextrin; cellulose derivatives such as crystalline cellulose; gum Arabic; dextran; or pullulan: or inorganic fillers including silicate derivatives such as light anhydrous silicic acid, synthetic aluminum silicate, calcium silicate or magnesium metasilicate aluminate; phosphates such as calcium hydrogenphosphate; carbonates such as calcium carbonate; or sulfates such as calcium sulfate. Of these, one or more fillers selected from cellulose derivatives and sugar derivatives are preferably used, one or more fillers selected from lactose, mannitol and crystalline cellulose are more preferably used, and one or more fillers selected from lactose and/or crystalline cellulose are most preferably used.

As the "lubricants" used, there may be mentioned for example, stearic acid; stearic acid metal salts such as calcium stearate or magnesium stearate; talc; colloidal silica; waxes such as beeswax or spermaceti; boric acid; adipic acid; sulfates such as sodium sulfate; glycol; fumaric acid; sodium stearyl fumarate; sucrose fatty acid esters; sodium benzoate; D,L-leucine; lauryl sulfates such as sodium lauryl sulfate or magnesium lauryl sulfate; silicates such as silicic anhydride or silicate hydrate; or the aforementioned starch derivatives. Of these, stearic acid metal salts are preferably used.

As the "binders" used, there may be mentioned for example, hydroxypropyl cellulose, hydroxypropylmethyl cellulose, polyvinylpyrrolidone, polyethylene glycol, or the compounds mentioned for the fillers. Of these, hydroxypropyl cellulose or hydroxypropylmethyl cellulose is preferably used.

As the "emulsifiers" used, there may be mentioned for example, colloidal clays such as bentonite or beegum; metal hydroxides such as magnesium hydroxide or aluminum hydroxide; anionic surfactants such as sodium lauryl sulfate or calcium stearate; cationic surfactants such as benzalkonium chloride; or nonionic surfactants such as polyoxyethylene alkyl ether, polyoxyethylene sorbitan fatty acid ester or sucrose fatty acid ester.

As the "stabilizers" used, there may be mentioned for example, para-oxybenzoic acid esters such as methyl paraben or propyl paraben; alcohols such as chlorobutanol, benzyl alcohol or phenyl ethyl alcohol; benzalkonium chloride; phenols such as phenol or cresol; thimerosal; dehydroacetic acid; or sorbic acid.

As the "corrigents" used, there may be mentioned for example, sweeteners such as sodium saccharin or aspartame; acidulants such as citric acid, malic acid or tartaric acid; or flavorings such as menthol, lemon or orange.

Although there is no particular limitation as regards the amount of the compound represented by the aforementioned general formula (I) or a pharmacologically acceptable salt thereof formulated in the entirety of the solid preparation, it is preferable to formulate 1.0 to 30.0 % by weight (preferably 1.3 to 20.0 % by weight) with respect to the total weight of the solid preparation.

Although there is no particular limitation as regards the amount of additives formulated in the entirety of the solid preparation, it is preferable to formulate 10.0 to 93.5 % by weight (preferably 44.0 to 90.0 % by weight) of fillers, 0.5 to 5.0 % by weight (preferably 0.5 to 3.0 % by weight) of lubricants, and 0.0 to 15.0 % by weight (preferably 2.5 to 10.0 % by weight) of binders with respect to the total weight of the solid preparation.

The "convection mixer equipped with a stirring blade" of the present invention will be explained hereinafter.

Mixers for mixing a granular material and the like can be roughly classified into "convection type" in which the mixing vessel is fixed and the particles to be mixed are moved by blades and airstreams, and "diffusion type" in which the mixing vessel performs rotational movement to move the particles. The "convection type" includes types in which a ribbon or a screw is rotated at slow speed such as PX Mixer, SV Mixer, Nauta Mixer, Ribbon Mixer, Loedige Mixer, Pug Mixer and the like (manufactured by Seishin Enterprise Co., Ltd., Shinko Pantec Co., Ltd., Hosokawa Micron Corporation, Tokuju Corporation, Matsubo Corporation, Fuji Paudal Co., Ltd, and the like), and "convection mixer equipped with a stirring blade" of the present invention in which a blade is rotated at high speed such as NMG, High-Speed Mixer, Vertical Granulator, Diosna, New Speed Kneader, Super Mixer, Henschel Mixer and the like (manufactured by Nara Machinery Co, Ltd., Fukae Powtec Co., Ltd., Powrex Corporation, Mutual Corporation, Okada Seiko Co., Ltd., Kawata Corporation, Mitsui Mining Co., Ltd., and the like). In the present invention, it is preferable to rotate the stirring blade such that the circumferential velocity at the end of the stirring blade is 1.4 m/s to 40 m/s (preferably 5.0 m/s to 40 m/s, and most preferably 5.0 m/s to 20 m/s).

As regards solid preparations of the present invention, there may be mentioned for example, tablets (including sublingual tablets and tablets that disintegrate in the mouth), capsules (including soft capsules and microcapsules), granules, fine granules, powders, pills, chewables or troches, preferably powders, fine granules, granules, capsules or tablets, and most preferably tablets.

As regards production methods for the solid preparation of the present invention, there may be used a general method described in publications such as "Powder Technology and Pharmaceutical Process (D. Chulia et al., Elservier Science Pub Co (December 1, 1993))". In particular, a dry method (for example, a dry granulation method or a direct compression method, preferably a direct compression method) is preferable.

In the direct compression method, the composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s or higher, followed by addition and mixing of lubricants and the like if necessary, and then the mixture is compression molded to produce a preparation.

The "dry granulation method" is a method in which a preparation is produced using granules prepared by mixing raw material powder by using the convection mixer equipped with a stirring blade, and then crushing and dividing by an appropriate method a compression-molded slug or sheet of the mixed raw material powders. These methods are described in publications such as "The Theory and Practice of Industrial Pharmacy (Third Edition) (Leon Lachman et al.: LEA & FEBIGER 1986)" and "Pharmaceutical Dosage Forms: Tablets volume 1 (Second Edition) (Herbert A. Lieberman et al.: MARCEL DEKKER INC. 1989)".

The compression-molding process is a process in which a mass product of raw material powder is formed by applying pressure to the raw material powder using mechanical force, and examples include rotary tableting machines (manufactured by Kikusui Seisakusho Ltd., Hata Iron Works Co., Ltd., Sugawara Seiki Co., Ltd. and the like), and dry granulators such as a roller compactor, a roll granulator and a Chilsonator (manufactured by Freund Corporation, Turbo Kogyo Co., Ltd., Kurimoto, Ltd., Matsubo Corporation, Nippon Granulator Co., Ltd., Fuji Paudal Co., Ltd. and the like).

The crushing and dividing process is a process in which the compressed mass formed in the compression-molding process is crushed by means of a knife or cutter into an appropriate size, and examples of apparatuses used include mills and particle size selectors such as a power mill, Fitzmill, Fiore, and Co-mill (manufactured by Fuji Paudal Co., Ltd., Tokuju Corporation, Powrex Corporation and the like).

The thus obtained granulated product is subjected to particle size regulation so as to have a desired particle diameter, and then a preparation in the form of powders, fine granules or granules is produced. These preparations can also be produced as capsules by packing them in a capsule, or can be produced as tablets by further adding disintegrants and/or lubricants if necessary and subjecting them to compression-molding by a tableting machine or the like. The operations of mixing and granulation are both widely used in the field of formulation techniques, and those skilled in the art can carry them out appropriately. In addition, tablets may be provided with at least one layer of a film-coating.

Coating is conducted by using a film-coating machine for example, and as the film coating base agent, there may be mentioned for example, sugar coating base agents, water-soluble film coating base agents, enteric film coating base agents or sustained release film coating base agents.

As the sugar coating base agents, saccharose is used, and it can be used in combination with one or more selected from talc, precipitated calcium carbonate, calcium phosphate, calcium sulfate, gelatin, gum Arabic, polyvinylpyrrolidone and pullulan.

As the water-soluble film coating base agents, there may be mentioned for example, cellulose derivatives such as hydroxypropyl cellulose, hydroxypropylmethyl cellulose, hydroxyethyl cellulose, methyl hydroxyethyl cellulose and sodium carboxymethyl cellulose; synthetic polymers such as polyvinyl acetal diethyl aminoacetate, aminoalkyl methacrylate copolymer and polyvinylpyrrolidone; and polysaccharides such as pullulan.

As the enteric film coating base agents, there may be mentioned for example, cellulose derivatives such as hydroxypropylmethyl cellulose phthalate, hydroxypropylmethyl cellulose acetate succinate, carboxymethylethyl cellulose or cellulose acetate phthalate; acrylic acid derivatives such as (meth)acrylic acid copolymer L, (meth)acrylic acid copolymer LD or (meth)acrylic acid copolymer S; or natural substances such as shellac.

As the sustained release film coating base agents, there may be mentioned for example, cellulose derivatives such as ethyl cellulose; or acrylic acid derivatives such as aminoalkyl methacrylate copolymer RS or ethyl acrylate-methyl methacrylate copolymer emulsion.

The aforementioned coating base agents may be used by combining two or more of them in an appropriate ratio. In addition, the coating base agents may, if necessary, further contain additives such as pharmacologically acceptable plasticizers, fillers, lubricants, masking agents, colorants and/or antiseptics.

The plasticizers which may be used in the present invention are not particularly limited, and a person skilled in the art can select them appropriately. As for such plasticizers, there may be mentioned for example, propylene glycol, polyethylene glycol, polypropylene glycol, glycerin and sorbitol, glycerin triacetate, diethyl phthalate and triethyl citrate, lauric acid, sucrose, dextrose, sorbitol, triacetin, acetyl triethyl citrate, triethyl citrate, tributyl citrate or acetyl tributyl citrate.

As the masking agents which may be used in the present invention, there may be mentioned for example, titanium oxide.

As the colorants which may be used in the present invention, there may be mentioned for example, titanium oxide, iron oxide, red ferric oxide, yellow ferric oxide or yellow No. 5 aluminum lake talc.

As the antiseptics which may be used in the present invention, there may be mentioned for example, paraben.

The dosage amount of the compound represented by the aforementioned general formula (I) or a pharmacologically acceptable salt thereof, which is an active ingredient of the pharmaceutical composition of the present invention, may vary depending on various conditions such as the activity of the drug, symptoms, age or body weight of a patient. The daily dosage amount for an adult human has a lower limit of 0.01 mg (preferably 1 mg) and an upper limit of 200 mg (preferably 100 mg) in the case of oral administration.

### [Examples]

The present invention will be described in more detail with reference to the Examples and Test Example; however, the present invention shall not be limited to these.

Here, "Compound A" used in the Examples is the compound represented by the following formula (Ia): 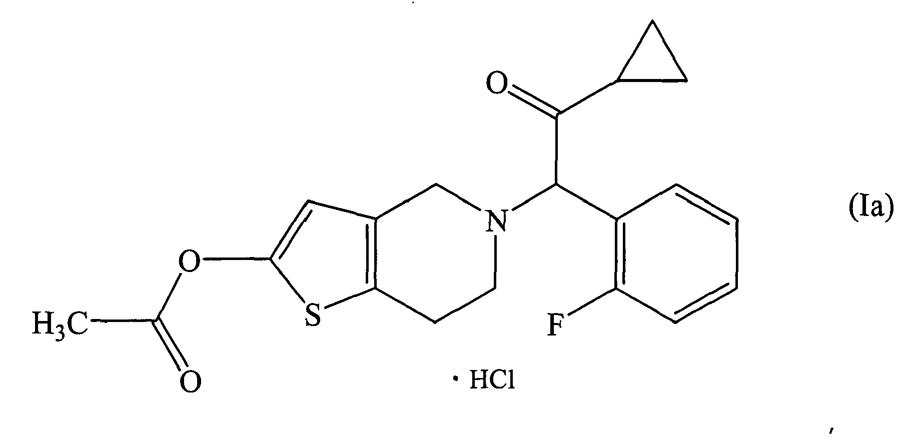 and can be prepared in accordance with the method disclosed in Japanese Patent Application (Kokai) No. 2002-145883.

### (Example 1)

Compound A (85.8 g), hydroxypropyl cellulose (125.0 g), croscarmellose sodium (125.0 g) and lactose (2139.0 g) were mixed for 3 minutes using a Henschel Mixer FM-20B (manufactured by Mitsui Miike Machinery CO., Ltd.) with the circumferential velocity at the end of the stirring blade being 14.1 m/s, followed by addition of magnesium stearate (25.0 g), and the mixture was mixed again for 15 seconds using the Henschel Mixer FM-20B (manufactured by Mitsui Miike Machinery CO., Ltd.) with the circumferential velocity at the end of the stirring blade being 14.1 m/s to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Dissolution testing was conducted on the obtained tablet. Test results are shown in Table 1.

### (Comparative Example 1)

Compound A (68.6 g), hydroxypropyl cellulose (100.0 g), croscarmellose sodium (100.0 g) and lactose (1711.0 g) were mixed for 30 minutes using a V-Type Mixer 10L (manufactured by Tokuju Corporation) which is a diffusion mixer, followed using the addition of magnesium stearate (20.0 g), and the mixture was mixed again using the V-Type Mixer 10L (manufactured by Tokuju Corporation) to give a mixed powder.

The mixed powder obtained was compressed using a rotary type tableting machine with a tableting pressure of 5.9 kN so that the tablet mass became approximately 80 mg. The uncoated tablet obtained was subjected to film-coating in a pan-coating machine, by spraying a coating solution consisting of hydroxypropylmethyl cellulose, lactose, titanium oxide, triacetin and water, to give a tablet containing the test compound. Dissolution testing was conducted on the obtained tablet. Test results are shown in Table 1.

### (Test Example 1) Dissolution Test

Testing was conducted in accordance with the Dissolution Test (Method 2) described in the 14^{th} Revised Edition of the Japanese Pharmacopoeia, using 900 mL of McIlvaine buffer (pH 4.4) as a test liquid at 50 revolutions per minute. A sample was individually taken from the test liquid after 5 minutes, 10 minutes, 15 minutes, and 30 minutes from the start of the test, and the dissolution rate of Compound A was measured by absorption spectrometry. (Dissolution tester, manufactured by Toyama Sangyo Co., Ltd., spectrophotometer, manufactured by Shimadzu Corporation.) The testing was conducted on 6 tablets, and the average value of the dissolution rate was calculated.

**(Table 1)**

| Dissolution Time (min) | Example 1 Dissolution rate (%) | Comparative Example 1 Dissolution rate (%) |
|---|---|---|
| 5 | 58 | 57 |
| 10 | 83 | 66 |
| 15 | 92 | 70 |
| 30 | 98 | 77 |

From Table 1, it is obvious that the preparation of Example 1 which is obtained through mixing by using a convection mixer with the circumferential velocity at the end of the stirring blade being 14.1 m/s, has excellent dissolvability compared with the preparation of Comparative Example 1 which is obtained through mixing by using a diffusion mixer.

### [INDUSTRIAL APPLICABILITY]

According to the present invention, a solid preparation containing the compound represented by the aforementioned general formula (I) or a pharmacologically acceptable salt thereof, which is produced by a method including a step in which a composition containing the compound represented by the aforementioned general formula (I) or a pharmacologically acceptable salt thereof is mixed while applying mechanical stress to the composition, has excellent dissolvability; in particular a solid preparation containing the compound represented by the aforementioned general formula (I) or a pharmacologically acceptable salt thereof, which is produced by a method including a step in which the composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s, has excellent dissolvability.

## Claims

1. A method for producing a solid preparation containing a compound represented by the following general formula (I): 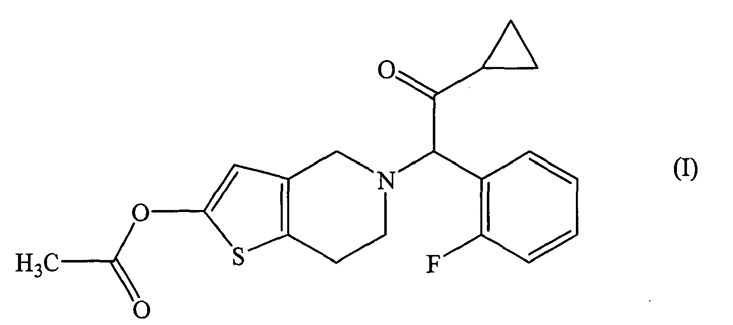 or a pharmacologically acceptable salt thereof, comprising a step wherein a composition containing the compound represented by the aforementioned general formula (I) or a pharmacologically acceptable salt thereof is mixed, while applying mechanical stress to the composition.

2. A method for producing a solid preparation containing a compound represented by the following general formula (I): 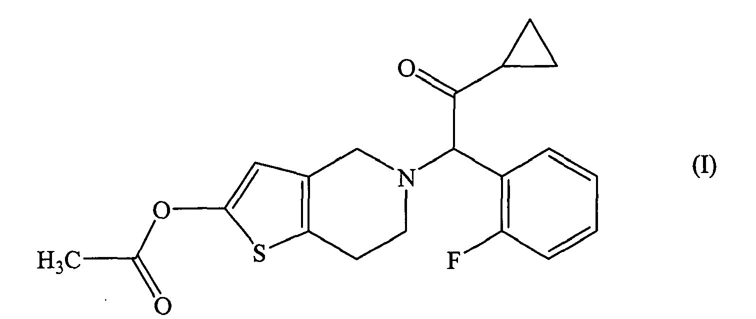 or a pharmacologically acceptable salt thereof, comprising a step wherein a composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s.

3. The method according to claim 2, wherein the circumferential velocity at the end of the stirring blade is 5.0 m/s to 20 m/s.

4. The solid preparation according to any one of claims 1 to 3, wherein the compound represented by the general formula (I) or a pharmacologically acceptable salt thereof is a compound represented by the following formula (Ia):

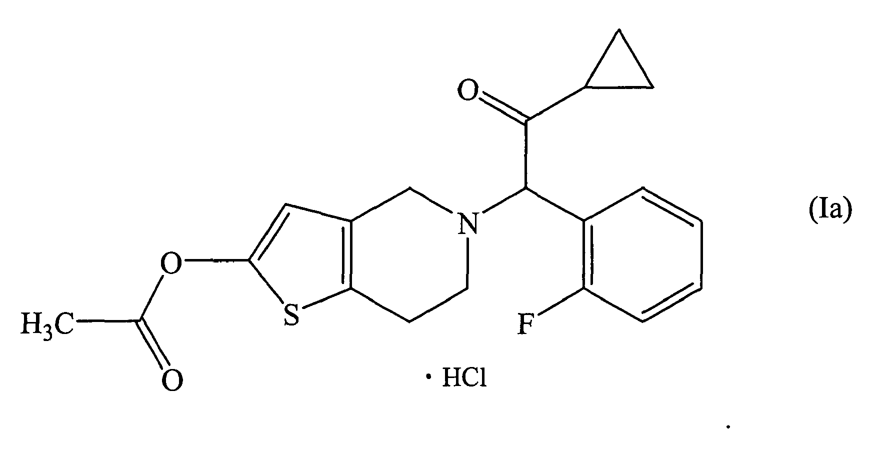

5. The method according to any one of claims 1 to 4, wherein the solid preparation is in the form of tablets.

6. A solid preparation containing a compound represented by the following general formula (I): 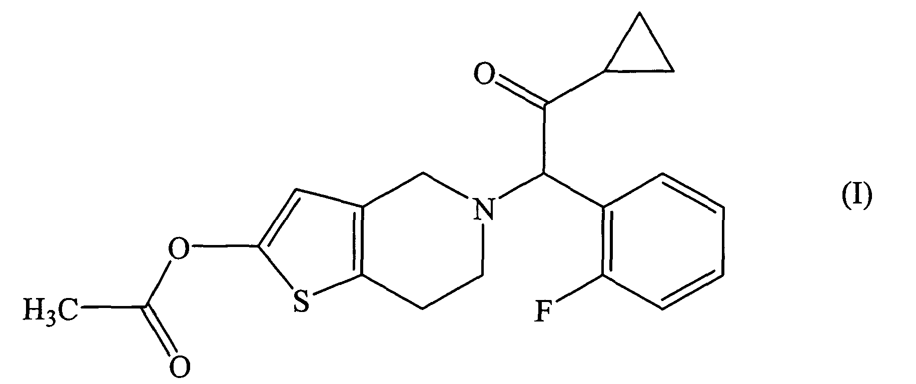 or a pharmacologically acceptable salt thereof, which is produced by a method comprising a step wherein a composition containing the compound represented by the aforementioned general formula (I) or a pharmacologically acceptable salt thereof is mixed, while applying mechanical stress to the composition.

7. A solid preparation containing a compound represented by the following general formula (I): 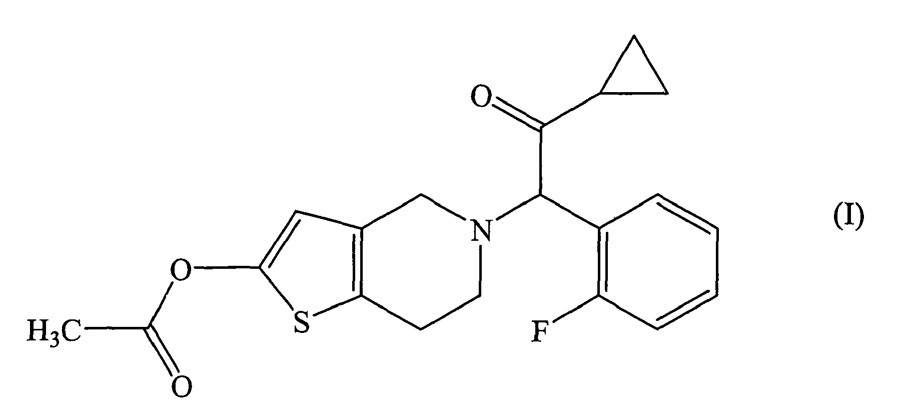 or a pharmacologically acceptable salt thereof, which is produced by a method comprising a step wherein a composition containing the compound represented by the aforementioned general formula (I) or a pharmacologically acceptable salt thereof is mixed by using a convection mixer equipped with a stirring blade and rotating the stirring blade such that the circumferential velocity at the end of the stirring blade is 1.4 m/s to 40 m/s.

8. The solid preparation according to claim 7, wherein the circumferential velocity at the end of the stirring blade is 5.0 m/s to 20 m/s.

9. The solid preparation according to any one of claims 6 to 8, wherein the compound represented by the general formula (I) or a pharmacologically acceptable salt thereof is a compound represented by the following formula (Ia):

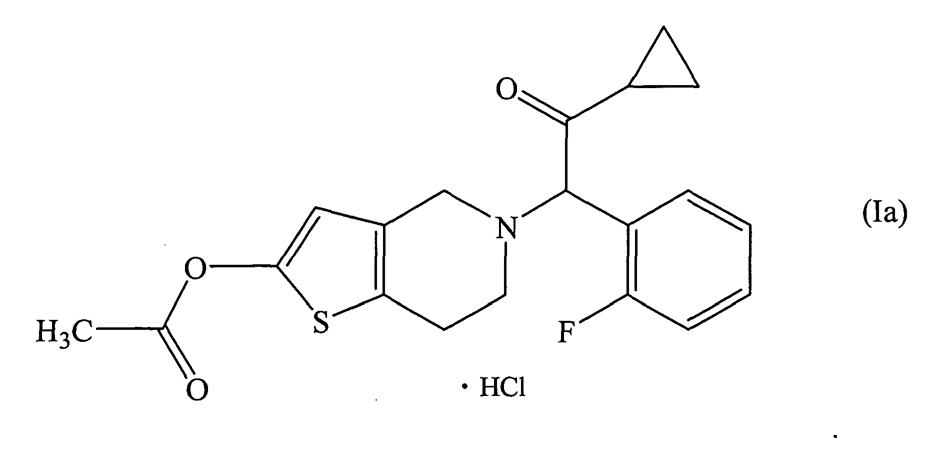

10. The solid preparation according to any one of claims 6 to 9, wherein the preparation is in the form of tablets.
